# EUROPÄISCHE PATENTSCHRIFT

(11) **EP 4 061 918 B1**
(45) Veröffentlichungstag und Bekanntmachung des Hinweises auf die Patenterteilung: **30.08.2023**
(21) Anmeldenummer: 20808066.3
(22) Anmeldetag: 16.11.2020
(51) Int. Cl.: C11D 11/00, C11D 3/39, C07D 223/10, C07D 207/27

(54) **BLEICHAKTIVATOR MIT KATIONISCHER GRUPPE UND DIESEN ENTHALTENDES WASCH- ODER REINIGUNGSMITTEL**
BLEACH ACTIVATOR HAVING A CATIONIC GROUP AND DETERGENT OR CLEANING PRODUCT CONTAINING SAME
ACTIVATEUR DE BLANCHIMENT AYANT UN GROUPE CATIONIQUE ET PRODUIT DÉTERGENT OU NETTOYANT LE CONTENANT

(30) Priorität: 20.11.2019 DE 102019217851
(43) Veröffentlichungstag der Anmeldung: 28.09.2022
(73) Patentinhaber: Henkel AG & Co. KGaA, 40589 Düsseldorf (DE)
(72) Erfinder: SCHAEFER, Sascha, 40822 Mettmann (DE); NEUHAUS, Svenja, 47179 Duisburg (DE); GEBERT-SCHWARZWAELDER, Antje, 41469 Neuss (DE); JUNKES, Christa, 40595 Düsseldorf (DE); JANSEN, Arne, 42781 Haan (DE)
(86) Internationale Anmeldenummer: PCT/EP2020/082198
(87) Internationale Veröffentlichungsnummer: WO 2021/099244

(56) Entgegenhaltungen:
- EP-A1- 0 816 336
- WO-A1-2017/102475
- WANG GUAN ET AL: "Synthesis, Characterization, and Toxicological Properties of New Cationic Bleach Activators", JOURNAL OF SURFACTANTS AND DETERGENTS, SPRINGER, BERLIN, DE, Bd. 20, Nr. 1, 14. November 2016 (2016-11-14), Seiten 277-285, XP036130379, ISSN: 1097-3958, DOI: 10.1007/S11743-016-1899-3 [gefunden am 2016-11-14] in der Anmeldung erwähnt

## Beschreibung

Die vorliegende Erfindung betrifft unter Perhydrolysebedingungen bestimmte kationische organische Persäurenden bildende Verbindungen, ihre Verwendung zur Aktivierung von Persauerstoffverbindungen in Zusammenhang mit dem Bleichen von Anschmutzungen beim Waschen von Textilien und Reinigen harter Oberflächen, sowie Wasch- und Reinigungsmittel, die sie enthalten. Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumcarbonat-Perhydrat, werden seit langem als Oxidationsmittel zu Desinfektions- und Bleichzwecken verwendet. Die Oxidationswirkung dieser Substanzen hängt in verdünnten Lösungen stark von der Temperatur ab; so erzielt man beispielsweise mit H₂O₂ oder Alkaliperborat in alkalischen Bleichflotten erst bei Temperaturen oberhalb von etwa 80 °C eine ausreichend schnelle Bleiche verschmutzter Textilien. Bei niedrigeren Temperaturen kann die Oxidationswirkung der anorganischen Persauerstoffverbindungen durch Zusatz sogenannter Bleichaktivatoren verbessert werden, die in der Lage sind, unter den angesprochenen Perhydrolysebedingungen Peroxocarbonsäuren zu liefern und für die zahlreiche Vorschläge, vor allem aus den Stoffklassen der N- oder O-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Hydrotriazine, Urazole, Diketopiperazine, Sulfurylamide und Cyanurate, außerdem Carbonsäureanhydride, insbesondere Phthalsäureanhydrid und Alkylbernsteinsäureanhydride, Carbonsäureester, insbesondere Natrium-nonanoyloxy-benzolsulfonat, Natrium-isononanoyloxy-benzolsulfonat, O-acylierte Zuckerderivate, wie Pentaacetylglukose, und N-acylierte Lactame, wie N-Benzoylcaprolactam, in der Literatur bekannt geworden sind. Durch Zusatz dieser Substanzen kann die Bleichwirkung wässriger Peroxidflotten so weit gesteigert werden, dass bereits bei Temperaturen um 60 °C im Wesentlichen die gleichen Wirkungen wie mit der Peroxidflotte allein bei 95 °C eintreten.

Im Bemühen um energiesparende Wasch- und Bleichverfahren gewinnen in den letzten Jahren Anwendungstemperaturen deutlich unterhalb 60 °C, insbesondere unterhalb 45 °C bis herunter zur Kaltwassertemperatur an Bedeutung.

Bei niedrigen Temperaturen lässt die Wirkung der bisher bekannten Aktivatorverbindungen in der Regel erkennbar nach.

Aus der internationalen Patentanmeldung WO 96/06915 A1 sind Bleichaktivatoren bekannt, die unter Perhydrolysebedingungen organische Peroxosäuren mit kationischer Gruppe bilden. EP0816336 A1 offenbart quartäre Ammoniumverbindungenals Bleichaktivatoren.

Überraschenderweise wurde nun gefunden, dass unter Perhydrolysebedingungen organische Peroxosäuren mit kationischer Gruppe bildende Verbindungen, bei denen ein quaternäres N-Atom Teil eines heterocyclischen 6-gliedrigen Restes ist, besonders geeignet sind, die Bleichwirkung von persauerstoffhaltigen Wasch- und Reinigungsmitteln zu verstärken.

Gegenstand der Erfindung ist daher eine bleichaktivierende Verbindung der allgemeinen Formel (I), 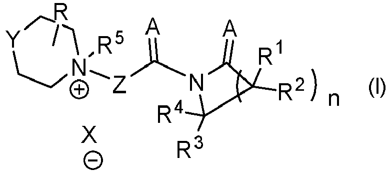 in der
A für O, S, Se;
Z für einen gegebenenfalls substituierten C₁₋₃₀ Alkylen-, C₃₋₃₀ Cycloalkylen-, Arylen-, Alkylenarylen-oder Arylenalkylen-Rest;
X⁻ für ein Anion, insbesondere Chlorid, Bromid, lodid, Tosylat, Mesylat, Triflat, Sulfat, Carbonat und/oder Phosphat;
Y für NR⁶, O, S, Se;
R, R¹, R², R³, R⁴, R⁵ und R⁶ unabhängig voneinander für H, einen C₁₋₃₀ Alkyl-, C₃₋₃₀ Cycloalkyl-, Aryl-, Alkylaryl- oder Arylalkyl-Rest; und
n für eine Zahl im Bereich von 1 bis 12 stehen.

In den angegebenen Kohlenwasserstoff-Resten können 1 oder mehrere nicht benachbarte, nicht an ein Heteroatom gebundene Kohlenstoffatome durch ein Heteroatom, insbesondere N, O, S und/oder Se, ausgetauscht sein. Falls X⁻ ein mehrwertiges Anion ist, sind entsprechend mehrere der den übrigen Teil der Verbindung gemäß Formel (I) bildenden, den kationischen Heterocyclus enthaltenden Einheiten pro Anion X⁻ vorhanden. R, R¹, R², R³, R⁴, R⁵ und/oder R⁶ sind vorzugsweise H, und/oder A ist vorzugsweise O, und/oder n ist vorzugsweise mindestens 2 und insbesondere eine Zahl im Bereich von 2 bis 4. Wie in der nachfolgenden Formel (II) wiedergegeben, leitet sich Z vorzugsweise von einer gegebenenfalls substituierten Alkylbenzol-Einheit ab, in der ein C-Atom des aromatischen Rings an die Einheit C=A gebunden ist, ein C-Atom des Alkylrestes an das gezeigte N-Atom des sechsgliedrigen Heterocyclus gebunden ist und die Methylgruppe der bevorzugte Alkylrest ist:

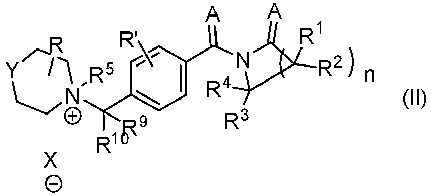

Für die Verbindungen der Formel (II) gelten die für Formel (I) angegebenen Definitionen für A, n, R und R¹ bis R⁵, Y und X⁻ ebenfalls; R', R⁹ und R¹⁰ stehen unabhängig voneinander für H, einen C₁₋₃₀ Alkyl-, C₃₋₃₀ Cycloalkyl-, Aryl-, Alkylaryl- oder Arylalkyl-Rest, wobei auch in diesen 1 oder mehrere nicht benachbarte, nicht an ein Heteroatom gebundene Kohlenstoffatome durch ein Heteroatom, insbesondere N, O, S und/oder Se, ausgetauscht sein können. Vorzugsweise sind R', R⁹ und/oder R¹⁰ H.

Ein weiterer Gegenstand der Erfindung ist die Verwendung von Verbindungen der allgemeinen Formel (I) zur Verstärkung der Reinigungsleistung von persauerstoffhaltigen und insbesondere tensidhaltigen Wasch- oder Reinigungsmitteln in wässriger Flotte.

Die erfindungsgemäße Verwendung besteht im Wesentlichen darin, Bedingungen zu schaffen, unter denen die Persauerstoffverbindung und die Verbindung der allgemeinen Formel (I) miteinander reagieren können, mit dem Ziel, stärker oxidierend wirkende Folgeprodukte zu erhalten. Solche Bedingungen liegen insbesondere dann vor, wenn die Reaktionspartner in einem wässrigen System aufeinandertreffen. Dies kann durch separate Zugabe der Persauerstoffverbindung und der Verbindung der allgemeinen Formel (I) zu einer gegebenenfalls wasch- oder reinigungsmittelhaltigen Flotte geschehen. Besonders vorteilhaft wird es jedoch unter Verwendung eines Wasch- oder Reinigungsmittels, welches die Verbindung der Formel (I) und ein peroxidisches Oxidationsmittel enthält, durchgeführt. Die Persauerstoffverbindung kann auch separat, in Substanz oder als vorzugsweise wässrige Lösung oder Suspension, zum wässrigen System zugegeben werden, wenn ein persauerstoffverbindungsfreies Wasch- oder Reinigungsmittel verwendet wird. Je nach Verwendungszweck können die Bedingungen weit variiert werden. So kommen neben rein wässrigen Lösungen auch Mischungen aus Wasser und geeigneten organischen Lösungsmitteln als Reaktionsmedium in Frage.

Weitere Gegenstände der Erfindung sind ein Verfahren zum Waschen von Wäsche und ein Verfahren zum Reinigen harter Oberflächen, umfassend die Verfahrensschritte (a) Bereitstellen einer wässrigen Flotte, enthaltend HzOz oder eine in Wasser HzOz liefernde, insbesondere anorganische Persauerstoffverbindung und eine Verbindung gemäß allgemeiner Formel (I), und (b) In-Kontakt-Bringen dieser Flotte mit zu waschenden Textilien oder zu reinigenden harten Oberflächen. Vorzugsweise stellt man den Kontakt zwischen der wässrigen Flotte und dem zu waschenden oder zu reinigenden Gegenstand bei Temperaturen im Bereich von 20 °C bis 40 °C, insbesondere von 20 °C bis 30 °C, her. Vorzugsweise bleibt der zu waschende oder zu reinigende Gegenstand über einen Zeitraum von 20 Minuten bis 120 Minuten, insbesondere von 30 Minuten bis 90 Minuten, mit der wässrigen Flotte in Kontakt. Die erfindungsgemäßen Verfahren können manuell oder mit Hilfe üblicher Vorrichtungen, beispielsweise Waschmaschinen oder Geschirrspülmaschinen, ausgeführt werden. Im Rahmen einer erfindungsgemäßen Verwendung und eines erfindungsgemäßen Verfahrens werden die Einsatzmengen an Persauerstoffverbindungen im Allgemeinen so gewählt, dass in der Flotte zwischen 10 ppm und 10 % Aktivsauerstoff, vorzugsweise zwischen 50 ppm und 5000 ppm Aktivsauerstoff vorhanden sind.

Eine Persauerstoffverbindung gemeinsam mit einer Verbindung gemäß allgemeiner Formel (I) wird vorzugsweise zum Bleichen von Anschmutzungen, insbesondere von Tee, beim Waschen von Textilien, insbesondere in wässriger, tensidhaltiger Flotte, verwendet. Die Formulierung "Bleichen von Anschmutzungen" ist dabei in ihrer weitesten Bedeutung zu verstehen und umfasst sowohl das Bleichen von sich auf dem Textil befindendem gefärbten Schmutz, das Bleichen von in der Waschflotte befindlichem, vom Textil abgelösten gefärbten Schmutz als auch das oxidative Zerstören von sich in der Waschflotte befindenden Textilfarben, die sich unter den Waschbedingungen von Textilien ablösen, bevor sie auf andersfarbige Textilien aufziehen können.

Eine weitere bevorzugte Anwendungsform gemäß der Erfindung ist die Verwendung einer Persauerstoffverbindung gemeinsam mit der Verbindung gemäß allgemeiner Formel (I) in insbesondere wässrigen, tensidhaltigen Reinigungslösungen für harte Oberflächen, insbesondere für Geschirr, zum Bleichen von gefärbten Anschmutzungen, zum Beispiel Tee. Auch dabei wird unter dem Begriff der Bleiche sowohl das Bleichen von sich auf der harten Oberfläche befindendem Schmutz, insbesondere Tee, als auch das Bleichen von in der Reinigungsflotte, zum Beispiel einer Geschirrspülflotte, befindlichem, von der harten Oberfläche abgelösten Schmutz verstanden.

Die erfindungsgemäßen Verwendungen zur Verstärkung der Reinigungsleistung von Wasch- und Reinigungsmitteln und zum Bleichen von gefärbten Anschmutzungen können besonders einfach dadurch realisiert werden, dass man die Verbindung der allgemeinen Formel (I) in eine insbesondere tensidhaltige wässrige Flotte einbringt, die auch Persauerstoffverbindung und das zu reinigende Textil oder den zu reinigenden Gegenstand mit harter Oberfläche enthält, wobei die Reihenfolge des Einbringens von Verbindung gemäß allgemeiner Formel (I), Persauerstoffverbindung und Textil oder Gegenstand mit harter Oberfläche beliebig ist, oder eine insbesondere tensidhaltige wässrige Flotte, welche die Verbindung der allgemeinen Formel (I) und Persauerstoffverbindung enthält, auf das zu reinigende Textil oder die zu reinigende harte Oberfläche aufbringt. Vorzugsweise wird die Verbindung der allgemeinen Formel (I) als Bestandteil eines Wasch- oder Reinigungsmittels eingesetzt, das besonders bevorzugt auch die Persauerstoffverbindung enthält. Weitere Gegenstände der Erfindung sind daher Wasch- oder Reinigungsmittel, enthaltend eine Verbindung gemäß allgemeiner Formel (l). Vorzugsweise enthält ein Wasch- oder Reinigungsmittel 0,01 Gew.-% bis 50 Gew.-%, insbesondere 0,1 Gew.-% bis 25 Gew.-% und besonders bevorzugt 1 Gew.-% bis 10 Gew.-% an Verbindung gemäß allgemeiner Formel (l). Die Angaben der Gew.-% beziehen sich dabei auf das gesamte Wasch- oder Reinigungsmittel; das gilt auch für alle folgenden Gew.-% - Angaben, wenn nicht ausdrücklich anders angegeben. In wässrigen Wasch- oder Reinigungsflotten werden vorzugsweise Mengen von 1 g/l bis 10 g/l, insbesondere von 2 g/l bis 5 g/l, solcher Mittel eingesetzt. Der pH-Wert der Wasch- und Reinigungsflotten ist in der Regel unkritisch und kann beispielsweise in Bereich von pH 4 bis pH 14 liegen; vorzugsweise liegt er im Bereich von pH 6 bis pH 12, insbesondere von pH 7 bis pH 10.

Die oben genannten bevorzugten Ausführungsformen der Verbindungen gemäß Formel (I) sind auch bevorzugt für den Einsatz in den erfindungsgemäßen Verfahren, Verwendungen und Mitteln.

Die Verbindungen der Formel (I) und die unter Perhydrolysebedingungen aus ihnen entstehenden Persäuren sind in der Regel besser wasserlöslich als analoge Verbindungen, in denen der kationische Substituent fehlt oder die stattdessen einen anionischen Substituenten tragen, und sie weisen eine höhere Affinität zu der zu reinigenden Oberfläche auf, insbesondere wenn diese aus Baumwolle besteht oder Baumwolle enthält.

Die erfindungsgemäßen Wasch- und Reinigungsmittel, die als insbesondere pulverförmige Feststoffe, in nachverdichteter Teilchenform, als homogene Lösungen oder als Suspensionen beziehungsweise Dispersionen vorliegen können, können außer der erfindungsgemäß verwendeten bleichverstärkenden Verbindung und vorzugsweise Persauerstoffverbindung im Prinzip alle bekannten und in derartigen Mitteln üblichen Inhaltsstoffe enthalten. Die erfindungsgemäßen Wasch-und Reinigungsmittel können insbesondere Buildersubstanzen, oberflächenaktive Tenside, wassermischbare organische Lösungsmittel, Enzyme, Sequestrierungsmittel, Elektrolyte, pH-Regulatoren und weitere Hilfsstoffe, wie optische Aufheller, Vergrauungsinhibitoren, Farbübertragungsinhibitoren, Schaumregulatoren, zusätzliche Persauerstoff-Aktivatoren sowie Farb- und Duftstoffe enthalten.

Als geeignete organische oder anorganische Persauerstoffverbindungen kommen organische Persäuren beziehungsweise persaure Salze organischer Säuren, wie Phthalimidopercapronsäure, Perbenzoesäure oder Salze der Diperdodecandisäure, insbesondere aber anorganische Persauerstoffverbindungen wie Wasserstoffperoxid und unter den Reinigungsbedingungen Wasserstoffperoxid abgebende anorganische Salze, wie Perborat, Percarbonat und/oder Persilikat, und Wasserstoffperoxid-Einschlussverbindungen, wie HzOz-Harnstoffaddukte, in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Die Persauerstoffverbindungen können als solche oder in Form diese enthaltender Mittel, die prinzipiell alle üblichen Wasch- oder Reinigungsmittelbestandteile enthalten können, zu der Wasch- beziehungsweise Reinigungslauge zugegeben werden. Besonders bevorzugt wird Alkalipercarbonat, Alkaliperborat-Monohydrat oder Wasserstoffperoxid in Form wässriger Lösungen, die 3 Gew.-% bis 10 Gew.-% Wasserstoffperoxid enthalten, eingesetzt. Hierbei und im Folgenden ist Natrium das bevorzugte Alkalimetall. Falls ein erfindungsgemäßes Wasch- oder Reinigungsmittel Persauerstoffverbindungen, insbesondere Natriumpercarbonat, enthält, sind diese vorzugsweise in Mengen von 1 Gew.-% bis 15 Gew.-%, insbesondere von 4 Gew.-% bis 6 Gew.-% darin vorhanden.

Als unter Perhydrolysebedingungen Peroxocarbonsäure-liefernde Verbindung können insbesondere Verbindungen, die unter Perhydrolysebedingungen gegebenenfalls substituierte Perbenzoesäure und/oder aliphatische Peroxocarbonsäuren mit 1 bis 12 C-Atomen, insbesondere 2 bis 4 C-Atomen ergeben, allein oder in Mischungen, eingesetzt werden. Geeignet sind die eingangs zitierten Bleichaktivatoren, die O- und/oder N-Acylgruppen insbesondere der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxo-hexahydro-1,3,5-triazin (DADHT), N-Acylimide, insbesondere N-Nonanoylsuccinimid (NOSI), acylierte Phenolsulfonate oder-carboxylate beziehungsweise die Sulfon- oder Carbonsäuren von diesen, insbesondere Nonanoyl- oder Isononanoyl- oder Lauroyloxybenzolsulfonat (NOBS beziehungsweise iso-NOBS beziehungsweise LOBS), 4-(2-Decanoyloxyethoxycarbonyloxy)-benzolsulfonat (DECOBS) oder Decanoyloxybenzoat (DOBA), acylierte mehrwertige Alkohole, insbesondere Triacetin, Ethylenglykoldiacetat und 2,5-Diacetoxy-2,5-dihydrofuran sowie acetyliertes Sorbitol und Mannitol und deren Mischungen (SORMAN), acylierte Zuckerderivate, insbesondere Pentaacetylglukose (PAG), Pentaacetylfruktose, Tetraacetylxylose und Octaacetyllactose, acetyliertes, gegebenenfalls N-alkyliertes Glucamin und Gluconolacton, und/oder N-acylierte Lactame, beispielsweise N-Benzoylcaprolactam. Ein erfindungsgemäßes Wasch- oder Reinigungsmittel ist vorzugsweise frei von weiteren unter Perhydrolysebedingungen Peroxocarbonsäure-liefernden Verbindungen.

Zusätzlich zu den genannten weiteren Verbindungen, die unter Perhydrolysebedingungen Peroxocarbonsäuren bilden, oder an deren Stelle können weitere bleichaktivierende Verbindungen, wie beispielsweise Nitrile, aus denen sich Perimidsäuren bilden, vorhanden sein. Dazu gehören insbesondere Aminoacetonitrilderivate mit quaterniertem Stickstoffatom gemäß der Formel 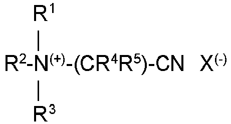 in der R¹ für -H, -CH₃, einen C₂₋₂₄-Alkyl- oder-Alkenylrest, einen substituierten C₁₋₂₄-Alkyl- oder C₂₋₂₄-Alkenylrest mit mindestens einem Substituenten aus der Gruppe -Cl, -Br, -OH, -NH₂, -CN und -N⁽⁺⁾-CH₂-CN, einen Alkyl- oder Alkenylarylrest mit einer C₁₋₂₄-Alkylgruppe, oder für einen substituierten Alkyl- oder Alkenylarylrest mit mindestens einer, vorzugsweise zwei, gegebenenfalls substituierten C₁₋₂₄-Alkylgruppe(n) und gegebenenfalls weiteren Substituenten am aromatischen Ring steht, R² und R³ unabhängig voneinander ausgewählt sind aus -CH₂-CN, -CH₃, -CH₂-CH₃, -CH₂-CH₂-CH₃, -CH(CH₃)-CH₃,-CH₂-OH, -CH₂-CH₂-OH, -CH(OH)-CH₃, -CH₂-CH₂-CH₂-OH, -CH₂-CH(OH)-CH₃, -CH(OH)-CH₂-CH₃, -(CH₂CH₂-O)ₙH mit n = 1, 2, 3, 4, 5 oder 6, R⁴ und R⁵ unabhängig voneinander eine voranstehend für R¹, R² oder R³ angegebene Bedeutung haben, wobei mindestens 2 der genannten Reste, insbesondere R² und R³, auch unter Einschluss des Stickstoffatoms und gegebenenfalls weiterer Heteroatome ringschließend miteinander verknüpft sein können und dann vorzugsweise einen Morpholino-Ring ausbilden, und X ein ladungsausgleichendes Anion, vorzugsweise ausgewählt aus Benzolsulfonat, Toluolsulfonat, Cumolsulfonat, den C₉₋₁₅-Al-kylbenzolsulfonaten, den C₁₋₂₀-Alkylsulfaten, den C₈₋₂₂-Carbonsäuremethylestersulfonaten, Sulfat, Hydrogensulfat und deren Gemischen, ist, können eingesetzt werden.

Auch die Anwesenheit von bleichkatalysierenden Übergangsmetallkomplexen ist möglich. Diese werden vorzugsweise unter den Cobalt-, Eisen-, Kupfer-, Titan-, Vanadium-, Mangan- und Rutheniumkomplexen ausgewählt. Als Liganden in den Übergangsmetallkomplexen kommen sowohl anorganische als auch organische Verbindungen in Frage, zu denen neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridylamin, Tris-(2-pyridylmethyl)amin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, N,N,N',N'-Tetrakis-(2-benzimi-dazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolylmethyl)aminomethyl)-benzol, Sorbitol, Mannitol, Erythritol, Adonitol, Inositol, Lactose, und gegebenenfalls substituierte Salene, Porphine und Porphyrine gehören. Zu den anorganischen Neutralliganden gehören insbesondere Ammoniak und Wasser. Falls nicht sämtliche Koordinationsstellen des Übergangsmetallzentralatoms durch Neutralliganden besetzt sind, enthält der Komplex weitere, vorzugsweise anionische und unter diesen insbesondere ein- oder zweizähnige Liganden. Zu diesen gehören insbesondere die Halogenide wie Fluorid, Chlorid, Bromid und lodid, und die (NO₂)⁻-Gruppe, das heißt ein Nitro-Ligand oder ein Nitrito-Ligand. Die (NO₂)⁻-Gruppe kann an ein Übergangsmetall auch chelatbildend gebunden sein oder sie kann zwei Übergangsmetallatome asymmetrisch oder η¹-O-verbrücken. Außer den genannten Liganden können die im Aktivatorsystem gemäß der Erfindung zu verwendenden Übergangsmetallkomplexe noch weitere, in der Regel einfacher aufgebaute Liganden, insbesondere ein- oder mehrwertige Anionliganden, tragen. In Frage kommen beispielsweise Nitrat, Acetat, Trifluoracetat, Formiat, Carbonat, Citrat, Perchlorat sowie komplexe Anionen wie Hexafluorophosphat. Die Anionliganden sollen für den Ladungsausgleich zwischen Übergangsmetall-Zentralatom und dem Ligandensystem sorgen. Auch die Anwesenheit von Oxo-Liganden, Peroxo-Liganden und Imino-Liganden ist möglich. Insbesondere derartige Liganden können auch verbrückend wirken, sodass mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome unterschiedliche Oxidationszahlen aufweisen, ist möglich. Falls Anionliganden fehlen oder die Anwesenheit von Anionliganden nicht zum Ladungsausgleich im Komplex führt, sind in den Übergangsmetallkomplex-Verbindungen anionische Gegenionen anwesend, die den kationischen Übergangsmetall-Komplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Benzoat, Citrat oder Oxalat. Beispiele für solche zusätzlichen Übergangsmetallkomplex-Verbindungen sind Mn(IV)₂(µ-O)s(1,4,7-trimethyl-1,4,7-triazacyclononan)-di-hexafluorophosphat, [N,N'-Bis[(2-hydroxy-5-vinylphe-nyl)-methylen]-1,2-diaminocyclohexan]-mangan-(lll)-chlorid, [N,N'-Bis[(2-hydroxy-5-nitrophenyl)-methylen]-1,2-diaminocyclohexan]-mangan-(lll)-acetat, [N,N'-Bis[(2-hydroxyphenyl)-methylen]-1,2-phenylendiamin]-mangan-(lll)-acetat, [N,N'-Bis[(2-hydroxyphenyl)-methylen]-1,2-diaminocyclohexan]-mangan-(lll)-chlorid, [N,N'-Bis[(2-hydroxyphenyl)-methylen]-1,2-diaminoethan]-mangan-(lll)-chlorid, [N,N'-Bis[(2-hydroxy-5-sulfonatophenyl)-methylen]-1,2-diaminoethan]-mangan-(lll)-chlorid, Mangan-Oxalat, Nitropentammin-cobalt(III)-chlorid, Nitritopentammin-cobalt(lll)-chlorid, Hexammin-cobalt(lll)-chlorid, Chloropentammin-cobalt(lll)-chlorid sowie der Peroxo-Komplex [(NH₃)₅Co-O-O-Co(NH₃)₅]Cl₄.

Die erfindungsgemäßen Mittel können ein oder mehrere Tenside enthalten, wobei insbesondere anionische Tenside, nichtionische Tenside und deren Gemische in Frage kommen. Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Derartige Tenside sind in den erfindungsgemäßen Reinigungs- oder Waschmitteln in Mengenanteilen von vorzugsweise 5 Gew.-% bis 50 Gew.-%, insbesondere von 8 Gew.-% bis 30 Gew.-%, enthalten, während die erfindungsgemäßen Mittel zur Reinigung von harten Oberflächen, insbesondere von Geschirr, vorzugsweise 0,1 Gew.-% bis 20 Gew.-%, insbesondere 0,2 Gew.-% bis 5 Gew.-% Tenside, enthalten.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure, Zuckersäuren und Carboxymethylinuline, monomere und polymere Aminopolycarbonsäuren, insbesondere Glycindiessigsäure, Methylglycindiessigsäure, Nitrilotriessigsäure, Iminodisuccinate wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylen-diamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden zugängliche Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative mittlere Molekülmasse (hier und im Folgenden: Gewichtsmittel) der Homopolymeren ungesättigter Carbonsäuren liegt im allgemeinen zwischen 5 000 g/mol und 200 000 g/mol, die der Copolymeren zwischen 2 000 g/mol und 200 000 g/mol, vorzugsweise 50 000 g/mol bis 120 000 g/mol, jeweils bezogen auf freie Säure. Ein besonders bevorzugtes Acrylsäure-Maleinsäure-Copolymer weist eine relative mittlere Molekülmasse von 50 000 g/mol bis 100 000 g/mol auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative mittlere Molekülmasse zwischen 1 000 g/mol und 200 000 g/mol, vorzugsweise zwischen 200 g/mol und 50 000 g/mol auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Als wasserlösliche anorganische Buildermaterialien kommen insbesondere Polyphosphate, vorzugsweise Natriumtriphosphat, in Betracht. Als wasserunlösliche anorganische Buildermaterialien werden insbesondere kristalline oder amorphe, wasserdispergierbare Alkalialumosilikate, in Mengen nicht über 25 Gew.-%, vorzugsweise von 3 Gew.-% bis 20 Gew.-% und insbesondere in Mengen von 5 Gew.-% bis 15 Gew.-% eingesetzt. Unter diesen sind die kristallinen Natriumalumosilikate in Waschmittelqualität, insbesondere Zeolith A, Zeolith P sowie Zeolith MAP und gegebenenfalls Zeolith X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen liegt in der Regel im Bereich von 100 bis 200 mg CaO pro Gramm.

Zusätzlich oder alternativ zum genannten wasserunlöslichen Alumosilikat und Alkalicarbonat können weitere wasserlösliche anorganische Buildermaterialien enthalten sein. Zu diesen gehören neben den Polyphosphaten wie Natriumtriphosphat insbesondere die wasserlöslichen kristallinen und/oder amorphen Alkalisilikat-Builder. Derartige wasserlösliche anorganische Buildermaterialien sind in erfindungsgemäßen Mitteln vorzugsweise in Mengen von 1 Gew.-% bis 20 Gew.-%, insbesondere von 5 Gew.-% bis 15 Gew.-% enthalten. Die als Buildermaterialien brauchbaren Alkalisilikate weisen vorzugsweise ein molares Verhältnis von Alkalioxid zu SiOz unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis NazO:SiOz von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ y H₂O eingesetzt, in der x, das sogenannte Modul, eine Zahl von 1,9 bis 4 und y eine Zahl von 0 bis 20 ist und bevorzugte Werte für x2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Natriumsilikate mit einem Modul im Bereich von 1,9 bis 3,5 werden in einer weiteren Ausführungsform erfindungsgemäßer Mittel eingesetzt. In einer bevorzugten Ausgestaltung erfindungsgemäßer Mittel setzt man ein granulares Compound aus Alkalisilikat und Alkalicarbonat ein, wie es zum Beispiel unter dem Namen Nabion^{®} 15 im Handel erhältlich ist.

Erfindungsgemäße maschinelle Geschirreinigungsmittel sind vorzugsweise niederalkalisch und enthalten die üblichen Alkaliträger wie zum Beispiel Alkalisilikate, Alkalicarbonate und/oder Alkalihydrogencarbonate. Zu den üblicherweise eingesetzten Alkaliträgern zählen Carbonate, Hydrogencarbonate und Alkalisilikate mit einem Molverhältnis SiO₂/M₂O (M = Alkaliatom) von 1,5 : 1 bis 2,5 : 1. Alkalisilikate können dabei in Mengen von bis zu 30 Gew.-%, bezogen auf das gesamte Mittel, enthalten sein. Auf den Einsatz der hoch alkalischen Metasilikate als Alkaliträger wird vorzugsweise ganz verzichtet. Das in den erfindungsgemäßen Mitteln bevorzugt eingesetzte Alkaliträgersystem ist ein Gemisch aus Carbonat und Hydrogencarbonat, vorzugsweise Natriumcarbonat und -hydrogencarbonat, das in einer Menge von bis zu 60 Gew.-%, vorzugsweise 10 Gew.-% bis 40 Gew.-%, enthalten ist. Je nachdem, welcher pH-Wert letztendlich gewünscht wird, variiert das Verhältnis von eingesetztem Carbonat und eingesetztem Hydrogencarbonat, üblicherweise wird jedoch ein Überschuss an Natriumhydrogencarbonat eingesetzt, sodass das Gewichtsverhältnis zwischen Hydrogencarbonat und Carbonat im Allgemeinen 1 : 1 bis 15 : 1 beträgt.

In einer weiteren Ausführungsform erfindungsgemäßer Mittel zur Reinigung von Geschirr sind in diesen 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Als in den erfindungsgemäßen Mitteln gegebenenfalls enthaltene Enzyme kommen insbesondere solche aus der Klasse der Proteasen, Lipasen, Cutinasen, Amylasen, Pullulanasen, Xylanasen, Hemicellulasen, Cellulasen, Peroxidasen sowie Oxidasen beziehungsweise deren Gemische in Frage, wobei der Einsatz von Protease, Amylase, Lipase und/oder Cellulase besonders bevorzugt ist. Der Anteil beträgt vorzugsweise 0,2 Gew.-% bis 1,5 Gew.-%, insbesondere 0,5 Gew.-% bis 1 Gew.-%. Die Enzyme können in üblicher Weise an Trägerstoffen adsorbiert und/oder in Hüllsubstanzen eingebettet sein oder als konzentrierte, möglichst wasserfreie Flüssigformulierungen eingearbeitet werden.

Geeignete Vergrauungsinhibitoren beziehungsweise soil-release-Wirkstoffe sind Celluloseether, wie Carboxymethylcellulose, Methylcellulose, Hydroxyalkylcellulosen und Cellulosemischether, wie Methylhydroxyethylcellulose, Methylhydroxypropylcellulose und Methyl-Carboxymethylcellulose. Vorzugsweise werden Natrium-Carboxymethylcellulose und deren Gemische mit Methylcellulose eingesetzt. Zu den üblicherweise eingesetzten Soil-release-Wirkstoffen gehören Copolyester, die Dicarbonsäureeinheiten, Alkylenglykoleinheiten und Polyalkylenglykoleinheiten enthalten. Der Anteil an Vergrauungsinhibitoren und/oder soil-release-Wirkstoffen in erfindungsgemäßen Mitteln liegt im Allgemeinen nicht über 2 Gew.-% und beträgt vorzugsweise 0,5 Gew.-% bis 1,5 Gew.-%.

Als optische Aufheller für insbesondere Textilien aus Cellulosefasern (zum Beispiel Baumwolle) können beispielsweise Derivate der Diaminostilbendisulfonsäure beziehungsweise deren Alkalimetallsalze enthalten sein. Geeignet sind zum Beispiel Salze der 4,4'-Bis(2-anilino-4-morpholino-1 ,3,5-triazin-6-yl-amino)-stilben-2,2'-disulfonsäure oder gleichartig aufgebaute Verbindungen, die anstelle der Morpholinogruppe eine Diethanolaminogruppe, eine Methylaminogruppe oder eine 2-Methoxyethylaminogruppe tragen. Weiterhin können Aufheller vom Typ des substituierten 4,4'-Distyryl-diphenyl anwesend sein, zum Beispiel 4,4'-Bis-(4-chlor-3-sulfostyryl)-diphenyl. Auch Gemische von Aufhellern können verwendet werden. Für Polyamidfasern eignen sich besonders gut Aufheller vom Typ der 1 ,3-Diaryl-2-pyrazoline, beispielsweise 1-(p-Sulfoamoylphenyl)-3-(p-chlorphenyl)-2-pyrazolin sowie gleichartig aufgebaute Verbindungen. Der Gehalt des Mittels an optischen Aufhellern beziehungsweise Aufhellergemischen liegt im Allgemeinen nicht über 1 Gew.-%, vorzugsweise 0,05 Gew.-% bis 0,5 Gew.-%. In einer bevorzugten Ausführungsform der Erfindung ist das Mittel frei von derartigen Wirkstoffen.

Zu den in den erfindungsgemäßen Mitteln einsetzbaren üblichen Schaumregulatoren gehören beispielsweise Polysiloxan-Kieselsäure-Gemische, wobei die darin enthaltene feinteilige Kieselsäure vorzugsweise silaniert oder anderweitig hydrophobiert ist. Die Polysiloxane können sowohl aus linearen Verbindungen wie auch aus vernetzten Polysiloxan-Harzen sowie aus deren Gemischen bestehen. Weitere Entschäumer sind Paraffinkohlenwasserstoffe, insbesondere Mikroparaffine und Paraffinwachse, deren Schmelzpunkt oberhalb 40 °C liegt, gesättigte Fettsäuren beziehungsweise Seifen mit insbesondere 20 bis 22 C-Atomen, zum Beispiel Natriumbehenat, und Alkalisalze von Phosphorsäuremono- und/oder -dialkylestern, in denen die Alkylketten jeweils 12 bis 22 C-Atome aufweisen. Unter diesen wird bevorzugt Natriummonoalkylphosphat und/oder -dialkylphosphat mit C₁₆- bis C₁₈-Alkylgruppen eingesetzt. Der Anteil der Schaumregulatoren kann vorzugsweise 0,2 Gew.-% bis 2 Gew.-% betragen.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in erfindungsgemäßen Mitteln in Mengen von vorzugsweise nicht über 20 Gew.-%, insbesondere von 1 Gew.-% bis 15 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Mitteln vorzugsweise nicht über 10 Gew.-%, insbesondere von 0,5 Gew.-% bis 6 Gew.-%, enthalten.

Die Herstellung der erfindungsgemäßen festen Mittel bietet keine Schwierigkeiten und kann in im Prinzip bekannter Weise, zum Beispiel durch Sprühtrocknen oder Granulation, erfolgen, wobei Persauerstoffverbindung und Bleichkatalysator gegebenenfalls später getrennt zugesetzt werden.

Erfindungsgemäße Mittel in Form wässriger oder sonstige übliche Lösungsmittel enthaltender Lösungen werden besonders vorteilhaft durch einfaches Mischen der Inhaltsstoffe, die in Substanz oder als Lösung in einen automatischen Mischer gegeben werden können, hergestellt.

Die erfindungsgemäßen Mittel liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung der thermisch belastbaren Komponenten und Zumischen der empfindlicheren Komponenten, zu denen insbesondere Enzyme, Bleichmittel und bleichaktivierende Verbindungen zu rechnen sind, hergestellt werden können. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt.

Zur Herstellung von Mitteln in Tablettenform geht man vorzugsweise derart vor, dass man alle Bestandteile in einem Mischer miteinander vermischt und das Gemisch mittels herkömmlicher Tablettenpressen, beispielsweise Exzenterpressen oder Rundläuferpressen, mit Pressdrucken im Bereich von 200 10⁵ Pa bis 1 500 · 10⁵ Pa verpresst. Man erhält so problemlos bruchfeste und dennoch unter Anwendungsbedingungen ausreichend schnell lösliche Tabletten mit Biegefestigkeit von normalerweise über 150 N. Vorzugsweise weist eine derart hergestellte Tablette ein Gewicht von 15 g bis 40 g, insbesondere von 20 g bis 30 g auf, bei einem Durchmesser von 35 mm bis 40 mm.

Die Herstellung erfindungsgemäßer Mittel in Form von nicht staubenden, lagerstabil rieselfähigen Pulvern und/oder Granulaten mit hohen Schüttdichten im Bereich von 800 g/l bis 1000 g/l kann auch dadurch erfolgen, dass man in einer ersten Verfahrensteilstufe die Builder-Komponenten mit wenigstens einem Anteil flüssiger Mischungskomponenten unter Erhöhung der Schüttdichte dieses Vorgemisches vermischt und nachfolgend - gewünschtenfalls nach einer Zwischentrocknung - die weiteren Bestandteile des Mittels, darunter den leistungsverstärkenden Wirkstoff oder die leistungsverstärkende Wirkstoffkombination, mit dem so gewonnenen Vorgemisch vereinigt.

### Beispiele

### Beispiel 1: Herstellung bleichverstärkender Verbindungen

a): Herstellung von N,N,N-Triethyl-N-(4-((2-oxoazepan-1-yl)carbonyl)phenyl)ethanaminiumchlorid (nicht erfindungsgemäß) (G. Wang, G. de Aragano Umbuzeiro, J. Aparecida Vendemiatti, A. Caloto de Oliveira, F. Inforcato Vacchi, M. Hussain, P. J. Hauser, H. S. Freeman, D. Hinks, J. Surfact. Deterg. 20 (2017), 277-285; S-H. Lim, N.Ç. Gürsoy, P. Hauser, D. Hinks, Color. Technol. 120 (2004), 114-118)
   a) i): Zu einer unter N₂-Atmosphäre zum Sieden unter Rückfluss erhitzten Lösung von 22,5 g Caprolactam (199 mmol) und 30,4 g Triethylamin (1,5 Äquivalente) in 250 ml Toluol wurde eine Lösung von 37,6 g 4-Chlormethyl-benzoylchlorid (199 mmol) in 20 ml Toluol zugetropft. Die Reaktionsmischung wurde unter Rühren 6 Stunden zum Sieden unter Rückfluss erhitzt, dann auf Raumtemperatur abkühlen lassen und filtriert. Das Filtrat wurde 12 Stunden lang im Kühlschrank aufbewahrt, der dabei ausgefallene Niederschlag wurde abfiltriert, mit 5%iger wässriger NaHCOs-Lösung gewaschen und 12 Stunden bei 40 °C getrocknet. Man erhielt 47,6 g (179 mmol; 90%) 1-((4-(Chlormethyl)phenyl)carbonyl)azepan-2-on als farblosen Feststoff.
      ¹H-NMR (400 MHz, DMSO-d₆): δ = 1,85 (bs, 6H), 2,67-2,73 (m, 2H), 3,94-4,01 (m, 2H), 4,59 (s, 2H), 7,41 (pd, J = 8,5 Hz, 2H), 7,53 (pd, J = 8,5 Hz, 2H) ppm.
   a) ii): Zu einer Lösung von 60 g von gemäß a) i) hergestelltem ((4-(Chlormethyl)phenyl)carbonyl)azepan-2-on (230 mmol) in 350 ml Acetonitril wurde unter N₂-Atmosphäre eine Lösung von 24,1 g Triethylamin (240 mmol) in 10 ml Acetonitril innerhalb von 1 Stunde zugetropft und die Reaktionsmischung wurde unter Rühren für 4 Stunden unter Rückfluss zum Sieden erhitzt. Anschließend wurde sie auf Raumtemperatur abgekühlt und das Lösungsmittel wurde am Rotationsverdampfer entfernt. Zum Rückstand wurden 40 ml Aceton gegeben, die Mischung wurde für 1 Stunde auf 50-60°C erhitzt, dann wurde das Aceton durch Filtrieren entfernt und der Vorgang dreimal wiederholt. Schließlich wurde der Rückstand bei 40 °C über Nacht getrocknet. Man erhielt 58,9 g (161 mmol; 72 %) N,N,N-Triethyl-N-(4-((2-oxoazepan-1-yl)carbonyl)phenyl)ethanaminium-chlorid als farbloses Pulver.
      ¹H-NMR (400 MHz, CDCl₃): δ = 1,37 (t, J = 7,2 Hz, 9H), 1,74-1,85 (bs, 6H), 2,60-2,68 (m, 2H), 3,35 (q, J = 7,2 Hz, 6H), 3,92 (bs, 2H), 4,82 (s, 2H), 7,47 (pd, J = 8,4 Hz, 2H), 7,63 (pd, J = 8,4 Hz, 2H) ppm.
b) Herstellung von 4-Methyl-4-(4-((2-oxoazepan-1-yl)carbonyl)benzyl)morpholin-4-iumchlorid Zu einer Lösung von 70 g von gemäß a) i) hergestelltem 1-((4-(Chlormethyl)phenyl)carbonyl)azepan-2-on (260 mmol) in 350 ml Acetonitril wurde unter N₂-Atmosphäre eine Lösung von 28 g N-Methylmorpholin (280 mmol) in 50 ml Acetonitril zugetropft und die Reaktionsmischung wurde unter Rühren für 4 Stunden unter Rückfluss zum Sieden erhitzt. Anschließend wurde sie auf Raumtemperatur abgekühlt und das Lösungsmittel wurde am Rotationsverdampfer entfernt. Zum Rückstand wurden 40 ml Aceton gegeben, die Mischung wurde für 1 Stunde auf 50-60°C erhitzt, dann wurde das Aceton durch Filtrieren entfernt und der Vorgang dreimal wiederholt. Schließlich wurde der Rückstand bei 40 °C über Nacht getrocknet. Man erhielt 79,1 g (216 mmol; 82%) 4-Methyl-4-(4-((2-oxoazepan-1-yl)carbonyl)benzyl)morpholin-4-iumchlorid als farblosen Feststoff.
c) Herstellung von 4-Methyl-4-(4-((2-oxopyrrolidin-1-yl)carbonyl)benzyl)morpholin-4-iumchlorid
   c) i): Zu einer unter N₂-Atmosphäre zum Sieden unter Rückfluss erhitzten Lösung von 34 g 2-Oxopyrrolidin (400 mmol) und 60,7 g Triethylamin (1,5 Äquivalente) in 450 ml Toluol wurde eine Lösung von 75,6 g 4-Chlormethyl-benzoylchlorid (400 mmol) in 50 ml Toluol zugetropft. Die Reaktionsmischung wurde unter Rühren 6 Stunden zum Sieden unter Rückfluss erhitzt, dann auf Raumtemperatur abkühlen lassen und filtriert. Das Filtrat wurde 12 Stunden lang im Kühlschrank aufbewahrt, der dabei ausgefallene Niederschlag wurde abfiltriert, mit 5%iger wässriger NaHCOs-Lösung gewaschen und 12 Stunden bei 40 °C getrocknet. Man erhielt 79,1,8 g (332 mmol; 83 %) 1-((4-(Chlormethyl)phenyl)carbonyl)pyrrolidin-2-on als farblosen Feststoff.
      ¹H-NMR (400 MHz, DMSO-d₆): δ = 1,75-1,95 (m, 4H, CH₂), 3,60-3,80 (m, 2H, CH₂), 4,81 (s, 2H, CH₂), 7,41-7,49 (m, 2H, Ar-H), 7,51-7,59 (m, 2H, Ar-H) ppm.
   c) ii) Zu einer Lösung von 22,7 g von gemäß c) i) hergestelltem 1-((4-(Chlormethyl)phenyl)carbonyl)pyrrolidin-2-on (95 mmol) in 125 ml Acetonitril wurde unter N₂-Atmosphäre eine Lösung von 10,1 g 1-N-Methylmorpholin (100 mmol) in 25 ml Acetonitril zugetropft und die Reaktionsmischung wurde unter Rühren für 4 Stunden unter Rückfluss zum Sieden erhitzt. Anschließend wurde sie auf Raumtemperatur abgekühlt und das Lösungsmittel wurde am Rotationsverdampfer entfernt. Zum Rückstand wurden 40 ml Aceton gegeben, die Mischung wurde für 1 Stunde auf 50-60°C erhitzt, dann wurde das Aceton durch Filtrieren entfernt und der Vorgang dreimal wiederholt. Schließlich wurde der Rückstand bei 40 °C über Nacht getrocknet. Man erhielt 25,6 g (79,8 mmol; 84 %) 4-Methyl-4-(4-((2-oxopyrrolidin-1-yl)carbonyl)benzyl)morpholin-4-iumchlorid als farblosen Feststoff.
      FTIR (film):ν̃ = 2966, 2889, 1734, 1661, 1480, 1415, 1358, 1300, 1245, 1189, 1114, 1065, 1019, 896, 862, 829, 750, 733, 631, 611, 553 cm⁻¹.
      ¹H-NMR (400 MHz, CDCl₃): δ = 2,06 (m, 2H), 2,54 (t, J = 4,0 Hz, 2H), 3,35 (s, 3H), 3,59 (d, J = 12,9 Hz, 2H), 3,70-3,80 (m, 2H), 3,84-4,03 (m, 4H), 3,89 (t, J = 7,1 Hz, 2H), 5,36 (s, 2H), 7,52 (pd, J = 8,3 Hz, 2H), 7,75 (pd, J = 8,3 Hz, 2H) ppm.
      ¹³C-NMR (100,6 MHz, D₂O): δ = 19,8 (t), 35,9 (t), 48,5 (q), 49,9 (t), 62,1 (t), 63,2 (t), 71,6 (t), 131,7 (d, 2C), 132,4 (s), 135,8 (d, 2C), 139,3 (s), 174,5 (s), 181,9 (s). MS (ESI positive mode, +3,5 kV): 234 (9%), 303 ([M-Cl⁻]⁺, 100%).

### Beispiel 2: Entfärbungsversuch

Zu einer durch Zugabe von NaOH auf pH 10-11 eingestellten Lösung von 2,5 mg Morin in 100 ml Wasser wurden 98 mg Natriumpercarbonat und jeweils 13,1 mg TAED oder einer der Verbindungen, die in den Beispielen 1 a) oder 1 b) hergestellt worden waren, hinzugefügt und bei Raumtemperaturgerührt. Die Zeitdauer bis zum vollständigen Verschwinden der gelben Farbe wurde bestimmt und ist in Tabelle 1 angegeben.

**Tabelle 1: Zeitdauer bis zur vollständigen Entfärbung**

| Wirkstoff | Dauer [Minuten] |
|---|---|
| TAED | 420 |
| Verbindung aus Beispiel 1 a) | 270 |
| Verbindung aus Beispiel 1 b) | 240 |
| Verbindung aus Beispiel 1 c) | 305 |

### Beispiel 3: Waschversuche

Es wurden Waschversuche bei 30 °C mit in Tabelle 3 angegebenen standardisierten Anschmutzungen auf Baumwolle unter Einsatz einer Waschlauge mit 3,8 g/ljeweils eines festen Waschmittels der in der nachfolgenden Tabelle 2 angegebenen Zusammensetzung durchgeführt, wobei die Mittel M1 und M2 die in Beispiel 1 b) und c) hergestellten erfindungsgemäßen Verbindungen, zum Vergleich das Mittel V2 die nicht erfindungsgemäße Verbindung aus Beispiel 1 a), und das Mittel V1 keine derartige Verbindung, sondern den herkömmlichen Bleichaktivator TAED enthielt. Die Auswertung erfolgte über Farbabstandsmessung gemäß der L*a*b*-Werte und die daraus berechneten Y-Werte als Maß für die Helligkeit. Tabelle 3 zeigt die Unterschiede der Y-Werte, die sich nach dem Waschen zwischen V1 und M1, V1 und M2 und V1 und V2 ergaben.

**Tabelle 2: Zusammensetzung (Gew.-%)**

| | V1 | M1 | M2 | V2 |
|---|---|---|---|---|
| Na-Alkylbenzolsulfonat | 13 | 13 | 13 | 13 |
| Na-Fettalkoholsulfat | 3 | 3 | 3 | 3 |
| Na-Carboxymethylcellulose | 3 | 3 | 3 | 3 |
| HEDP-Na4 | 1 | 1 | 1 | 1 |
| Na-Polyacrylat | 3 | 3 | 3 | 3 |
| Natriumsilicat | 7 | 7 | 7 | 7 |
| Natriumcarbonat | 21 | 21 | 21 | 21 |
| Natriumhydrogencarbonat | 10 | 10 | 10 | 10 |
| Natriumpercarbonat | 12 | 12 | 12 | 12 |
| TAED | 3,1 | - | - | - |
| Verbindung aus Beispiel 1 c) | - | 3,1 | - | - |
| Verbindung aus Beispiel 1 b) | - | - | 3,1 | - |
| Verbindung aus Beispiel 1 a) | - | - | - | 3,1 |
| Wasser, Natriumsulfat, Parfum, Farbstoff | auf 100 | | | |

**Tabelle 3: ΔY-Werte**

| Anschmutzung / Mittel | M1 | M2 | V2 |
|---|---|---|---|
| Rotwein | 3,9 | 4,5 | n.b. |
| Kaffee | 2,3 | 3,4 | n.b. |
| Rote Beete | 2,7 | 2,6 | n.b. |
| Tee | 2,4 | 1,8 | 1,5 |
| Kirschsaft | 2,4 | 2,0 | n.b. |
| Apfel | 1,8 | 1,7 | 1,0 |

| | | | |
|---|---|---|---|
| n.b.: nicht bestimmt | | | |

Die Helligkeitswerte bei Einsatz der erfindungswesentlichen Wirkstoffe sind größer als diejenigen, die sich beim Einsatz der andere Bleichaktivatoren enthaltenden Waschmittel ergeben.

### Beispiel 4: Reinigungsversuche

0,5 g einer Mischung von Lactose und Ovalbumin (Gewichtsverhältnis 3:1) wurden in 10 ml Wasser bei Raumtemperatur 10 Minuten lang gerührt, dann wurde die Mischung gefriergetrocknet, in einem Mörser gemahlen und 20 Minuten lang auf 160 °C erhitzt. Mischungen von jeweils 15 mg des so erhaltenen, angebrannte Nahrungsmittelrückstände simulierenden Schmutzes, 3,75 mg Natriumpercarbonat und 0,5 mg einer in Beispiel 1 a), 1 b) oder 1 c) hergestellten Verbindung in 5 ml Wasser wurden 48 Stunden lang in einer mechanischen Schüttelvorrichtung auf 60 °C erwärmt. Nach den in Tabelle 4 angegebenen Zeitdauern wurde die Auflösung des künstlichen Schmutzes visuell ausgewertet und auf einer Skala von 0 (keine Auflösung) bis ++++ (vollständig aufgelöst) benotet.

**Tabelle 4: Auflösung von angebranntem Schmutz**

| Wirkstoff / Zeitdauer | 0,5h | 1h | 2h | 4h | 6h | 24h | 48h |
|---|---|---|---|---|---|---|---|
| kein | 0 | 0 | 0 | 0 | 0 | 0 | 0 |
| Verbindung aus Beispiel 1 b) | + | + | ++ | +++ | +++ | +++ | ++++ |
| Verbindung aus Beispiel 1 c) | + | + | ++ | +++ | +++ | +++ | ++++ |
| Verbindung aus Beispiel 1 a) | 0 | 0 | + | + | + | + | ++ |

### Beispiel 5: Weiterer Reinigungsversuch

In einer Geschirrspülmaschine Miele^{®} GSL2 wurde im Programm 45 8 55 unter Einsatz von Wasser mit einer Wasserhärte 21 °dH und einer natriumpercarbonathaltigen und bleichaktivator- und - katalysatorfreien Geschirrspülmitteltablette, der man zum Vergleich 0,4 g TAED (Mittel V3) oder 0,59 g der Verbindung aus Beispiel 1 a) (Mittel V4), oder 0,55 g der Verbindung aus Beispiel 1 c) (Mittel M3) oder 0,58 g der Verbindung aus Beispiel 1 b) (Mittel M4) zugefügt hatte, mit den in der nachfolgenden Tabelle 5 angegebenen standardisierten Anschmutzungen versehenes Geschirr gespült. Die Schmutzentfernungsleistung wurde von geschulten Beobachtern optisch begutachtet und anhand der in SÖFW-Journal 132, 2006, 35-49 veröffentlichten Vergleichsabbildungen auf einer Skala von 0 (= stark verschmutzt) bis 10 (= vollständig sauber) benotet. Es ergaben sich die ebenfalls in Tabelle 5 angegebenen Noten für die die bleichaktivatoren in etwa molgleicher Menge enthaltenden Mittel.

**Tabelle 5: Reinigungsnoten**

| Mittel /Anschmutzung | Tee (Assam) | Tee (BOP) | Crème Brûlèe | Eingebranntes Hackfleisch | Eigelb | Stärke |
|---|---|---|---|---|---|---|
| V3 | 2,0 | 2,0 | 6,6 | 4,8 | 3,2 | 5,9 |
| V4 | 6,2 | 6,7 | 6,5 | 5,6 | 3,1 | 6,2 |
| M3 | 7,2 | 7,0 | 7,0 | 5,3 | n.b. | 6,6 |
| M4 | 6,8 | n.b. | n.b. | 6,0 | 3,4 | n.b. |

| | | | | | | |
|---|---|---|---|---|---|---|
| n.b.: nicht bestimmt | | | | | | |

Man erkennt, dass die Mittel mit den erfindungsgemäßen Wirkstoffen besser abschnitten als die zum Vergleich getesteten Mittel.

## Patentansprüche

1. Bleichaktivierende Verbindung der allgemeinen Formel (l), 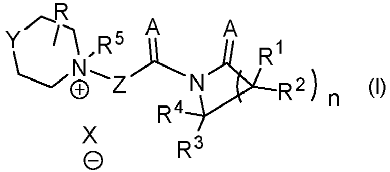 in der
A für O, S, Se;
Z für einen gegebenenfalls substituierten C₁₋₃₀ Alkylen-, C₃₋₃₀ Cycloalkylen-, Arylen-, Alkylenarylen- oder Arylenalkylen-Rest;
X⁻ für ein Anion, insbesondere Chlorid, Bromid, lodid, Tosylat, Mesylat, Triflat, Sulfat, Carbonat und/oder Phosphat;
Y für NR⁶, O, S, Se;
R, R¹, R², R³, R⁴, R⁵ und R⁶ unabhängig voneinander für H, einen C₁₋₃₀ Alkyl-, C₃₋₃₀ Cycloalkyl-, Aryl-, Alkylaryl- oder Arylalkyl-Rest; und
n für eine Zahl im Bereich von 1 bis12 stehen,
wobei in den Kohlenwasserstoff-Resten 1 oder mehrere nicht benachbarte, nicht an ein Heteroatom gebundene Kohlenstoffatome durch ein Heteroatom, insbesondere N, O, S und/oder Se, ausgetauscht sein können.

2. Verwendung von Verbindungen der allgemeinen Formel (l), 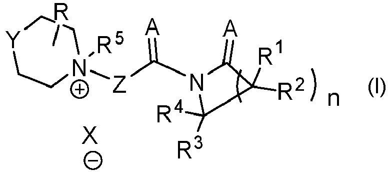 in der
A für O, S, Se;
Z für einen gegebenenfalls substituierten C₁₋₃₀ Alkylen-, C₃₋₃₀ Cycloalkylen-, Arylen-, Alkylenarylen- oder Arylenalkylen-Rest;
X⁻ für ein Anion, insbesondere Chlorid, Bromid, lodid, Tosylat, Mesylat, Triflat, Sulfat, Carbonat und/oder Phosphat;
Y für NR⁶, O, S, Se;
R, R¹, R², R³, R⁴, R⁵ und R⁶ unabhängig voneinander für H, einen C₁₋₃₀ Alkyl-, C₃₋₃₀ Cycloalkyl-, Aryl-, Alkylaryl- oder Arylalkyl-Rest; und
n für eine Zahl im Bereich von 1 bis12 stehen;
wobei in den Kohlenwasserstoff-Resten 1 oder mehrere nicht benachbarte, nicht an ein Heteroatom gebundene Kohlenstoffatome durch ein Heteroatom, insbesondere N, O, S und/oder Se, ausgetauscht sein können,
zur Verstärkung der Reinigungsleistung von persauerstoffhaltigen und insbesondere tensidhaltigen Wasch- oder Reinigungsmitteln in wässriger Flotte.

3. Verwendung nach Anspruch 2, **dadurch gekennzeichnet, dass** man die Verbindung der allgemeinen Formel (I) in eine insbesondere tensidhaltige wässrige Flotte einbringt, die auch Persauerstoffverbindung und das zu reinigende Textil oder den zu reinigenden Gegenstand mit harter Oberfläche enthält, wobei die Reihenfolge des Einbringens von Verbindung gemäß allgemeiner Formel (I), Persauerstoffverbindung und Textil oder Gegenstand mit harter Oberfläche beliebig ist, oder eine insbesondere tensidhaltige wässrige Flotte, welche die Verbindung der allgemeinen Formel (I) und Persauerstoffverbindung enthält, auf das zu reinigende Textil oder die zu reinigende harte Oberfläche aufbringt.

4. Verfahren zum Waschen von Wäsche oder Verfahren zum Reinigen harter Oberflächen, umfassend die Verfahrensschritte
(a) Bereitstellen einer wässrigen Flotte, enthaltend HzOz oder eine in Wasser HzOz liefernde, insbesondere anorganische Persauerstoffverbindung und eine Verbindung gemäß allgemeiner Formel (l), 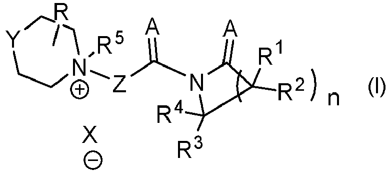 in der
A für O, S, Se;
Z für einen gegebenenfalls substituierten C₁₋₃₀ Alkylen-, C₃₋₃₀ Cycloalkylen-, Arylen-, Alkylenarylen- oder Arylenalkylen-Rest;
X⁻ für ein Anion, insbesondere Chlorid, Bromid, lodid, Tosylat, Mesylat, Triflat, Sulfat, Carbonat und/oder Phosphat;
Y für NR⁶, O, S, Se;
R, R¹, R², R³, R⁴, R⁵ und R⁶ unabhängig voneinander für H, einen C₁₋₃₀ Alkyl-, C₃₋₃₀ Cycloalkyl-, Aryl-, Alkylaryl- oder Arylalkyl-Rest; und
n für eine Zahl im Bereich von 1 bis12 stehen;
wobei in den Kohlenwasserstoff-Resten 1 oder mehrere nicht benachbarte, nicht an ein Heteroatom gebundene Kohlenstoffatome durch ein Heteroatom, insbesondere N, O, S und/oder Se, ausgetauscht sein können, und
(b) In-Kontakt-Bringen dieser Flotte mit zu waschenden Textilien oder zu reinigenden harten Oberflächen.

5. Verfahren nach Anspruch 4, **dadurch gekennzeichnet, dass** man den Kontakt zwischen der wässrigen Flotte und dem zu waschenden oder zu reinigenden Gegenstand bei Temperaturen im Bereich von 20 °C bis 40 °C, insbesondere von 20 °C bis 30 °C, herstellt.

6. Verfahren nach Anspruch 4 oder 5, **dadurch gekennzeichnet, dass** der zu waschende oder zu reinigende Gegenstand über einen Zeitraum von 20 Minuten bis 120 Minuten, insbesondere von 30 Minuten bis 90 Minuten, mit der wässrigen Flotte in Kontakt bleibt.

7. Verwendung nach Anspruch 2 oder 3 oder Verfahren nach einem der Ansprüche 4 bis 6,
**dadurch gekennzeichnet, dass** man die Einsatzmengen an Persauerstoffverbindungen so wählt, dass in der Flotte zwischen 10 ppm und 10 % Aktivsauerstoff, vorzugsweise zwischen 50 ppm und 5000 ppm Aktivsauerstoff vorhanden sind.

8. Wasch- oder Reinigungsmittel, enthaltend eine Verbindung gemäß allgemeiner Formel (l), 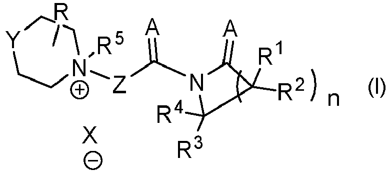 in der
A für O, S, Se;
Z für einen gegebenenfalls substituierten C₁₋₃₀ Alkylen-, C₃₋₃₀ Cycloalkylen-, Arylen-, Alkylenarylen- oder Arylenalkylen-Rest;
X⁻ für ein Anion, insbesondere Chlorid, Bromid, lodid, Tosylat, Mesylat, Triflat, Sulfat, Carbonat und/oder Phosphat;
Y für NR⁶, O, S, Se;
R, R¹, R², R³, R⁴, R⁵ und R⁶ unabhängig voneinander für H, einen C₁₋₃₀ Alkyl-, C₃₋₃₀ Cycloalkyl-, Aryl-, Alkylaryl- oder Arylalkyl-Rest; und
n für eine Zahl im Bereich von 1 bis12 stehen;
wobei in den Kohlenwasserstoff-Resten 1 oder mehrere nicht benachbarte, nicht an ein Heteroatom gebundene Kohlenstoffatome durch ein Heteroatom, insbesondere N, O, S und/oder Se, ausgetauscht sein können.

9. Mittel nach Anspruch 8, **dadurch gekennzeichnet, dass** es 0,01 Gew.-% bis 50 Gew.-%, insbesondere 0,1 Gew.-% bis 25 Gew.-% an Verbindung gemäß allgemeiner Formel (I) enthält.

10. Verwendung, Mittel oder Verfahren nach einem der voranstehenden Ansprüche, **dadurch gekennzeichnet, dass** die Verbindung der allgemeinen Formel (I) der allgemeinen Formel (II) entspricht, 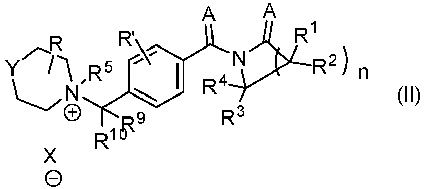 in der R`, R⁹ und R¹⁰ unabhängig voneinander für H, einen C₁₋₃₀ Alkyl-, C₃₋₃₀ Cycloalkyl-, Aryl-, Alkylaryl- oder Arylalkyl-Rest stehen, wobei in diesen 1 oder mehrere nicht benachbarte, nicht an ein Heteroatom gebundene Kohlenstoffatome durch ein Heteroatom, insbesondere N, O, S und/oder Se, ausgetauscht sein können.

## Claims

1. A bleach-activating compound of the general formula (l), 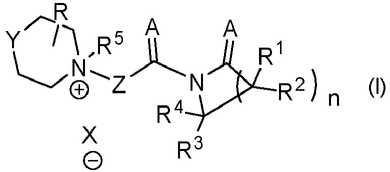 in which
A stands for O, S, Se;
Z represents an optionally substituted C₁₋₃₀ alkylene, C₃₋₃₀ cycloalkylene, arylene, alkylene arylene or arylene alkylene radical;
X⁻ stands for an anion, in particular chloride, bromide, iodide, tosylate, mesylate, triflate, sulphate, carbonate and/or phosphate;
Y stands for NR⁶ , O, S, Se;
R, R¹, R², R³, R⁴, R⁵ and R⁶ are independently H, a C₁₋₃₀ alkyl, C₃₋₃₀ cycloalkyl, aryl, alkylaryl or arylalkyl radical; and
n stands for a number in the range from 1 to12,
wherein 1 or more non-adjacent carbon atoms not bonded to a heteroatom in the hydrocarbon radicals may be replaced by a heteroatom, in particular N, O, S and/or Se.

2. Use of compounds of the general formula (l), 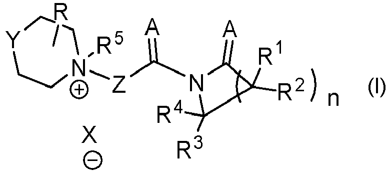 in which
A stands for O, S, Se;
Z represents an optionally substituted C₁₋₃₀ alkylene, C₃₋₃₀ cycloalkylene, arylene, alkylene a rylene or arylenealkylene radical;
X⁻ stands for an anion, in particular chloride, bromide, iodide, tosylate, mesylate, triflate, sulphate, carbonate and/or phosphate;
Y stands for NR⁶, O, S, Se;
R, R¹, R², R³, R⁴, R⁵ and R⁶ are independently H, a C₁₋₃₀ alkyl, C₃₋₃₀ cycloalkyl, aryl, alkylaryl or arylalkyl radical; and
n stands for a number in the range from 1 to12;
wherein 1 or more non-adjacent carbon atoms not bonded to a heteroatom in the hydrocarbon radicals may be replaced by a heteroatom, in particular N, O, S and/or Se,
for enhancing the cleaning performance of peroxygen-containing and in particular surfactant containing detergents or cleaning agents in aqueous liquor.

3. Use according to claim 2, **characterized in that** the compound of the general formula (l) is introduced into an aqueous liquor, in particular one containing surfactants, which also contains peroxygen compound and the textile or hard-surface object to be cleaned, the order of introduction of the compound according to the general formula (I), peroxygen compound and textile or hard-surface object to be cleaned being arbitrary, peroxygen compound and textile or hard-surface article is arbitrary, or an aqueous liquor, in particular one containing surfactants, which contains the compound of the general formula (l) and peroxygen compound, is applied to the textile or hard surface to be cleaned.

4. A method of washing laundry or a method of cleaning hard surfaces comprising the steps of
(a) Providing an aqueous liquor containing H O₂₂ or a peroxygen compound, in particular an inorganic peroxygen compound, yielding H O₂₂ in water and a compound according to general formula (l), 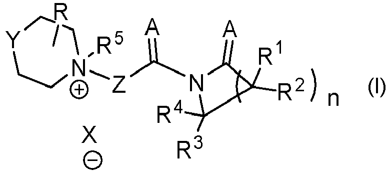 in which
A stands for O, S, Se;
Z represents an optionally substituted C₁₋₃₀ alkylene, C₃₋₃₀ cycloalkylene, arylene, alkylene a rylene or arylenealkylene radical;
X⁻ stands for an anion, in particular chloride, bromide, iodide, tosylate, mesylate, triflate, sulphate, carbonate and/or phosphate;
Y stands for NR⁶, O, S, Se;
R, R¹, R², R³, R⁴, R⁵ and R⁶ are independently H, a C₁₋₃₀ alkyl, C₃₋₃₀ cycloalkyl, aryl, alkylaryl or arylalkyl radical; and
n stands for a number in the range from 1 to12;
wherein 1 or more non-adjacent carbon atoms not bonded to a heteroatom in the hydrocarbon radicals may be replaced by a heteroatom, in particular N, O, S and/or Se, and
(b) Bringing the liquor into contact with textiles to be washed or hard surfaces to be cleaned.

5. The method according to claim 4, **characterized in that** the contact between the aqueous liquor and the object to be washed or cleaned is established at temperatures in the range from 20 °C to 40 °C, in particular from 20 °C to 30 °C.

6. Process according to claim 4 or 5, **characterized in that** the object to be washed or cleaned remains in contact with the aqueous liquor for a period of from 20 minutes to 120 minutes, in particular from 30 minutes to 90 minutes.

7. Use according to claim 2 or 3, or process according to any of claims 4 to 6, **characterized in that** the input quantities of peroxygen compounds are chosen such that between 10 ppm and 10 % active oxygen, preferably between 50 ppm and 5000 ppm active oxygen, are present in the liquor.

8. A detergent or cleaning composition comprising a compound according to general formula (l), 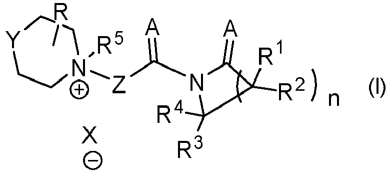 in which
A stands for O, S, Se;
Z represents an optionally substituted C₁₋₃₀ alkylene, C₃₋₃₀ cycloalkylene, arylene, alkylene a rylene or arylenealkylene radical;
X⁻ stands for an anion, in particular chloride, bromide, iodide, tosylate, mesylate, triflate, sulphate, carbonate and/or phosphate;
Y stands for NR⁶ , O, S, Se;
R, R¹, R², R³, R⁴, R⁵ and R⁶ are independently H, a C₁₋₃₀ alkyl, C₃₋₃₀ cycloalkyl, aryl, alkylaryl or arylalkyl radical; and
n stands for a number in the range from 1 to12;
wherein 1 or more non-adjacent carbon atoms not bonded to a heteroatom in the hydrocarbon radicals may be replaced by a heteroatom, in particular N, O, S and/or Se.

9. Composition according to claim 8, **characterized in that** it contains 0.01 wt.% to 50 wt.%, in particular 0.1 wt.% to 25 wt.%, of compound according to general formula (I).

10. Use, composition or method according to any one of the preceding claims, **characterized in that** the compound of general formula (l) corresponds to general formula (ll), 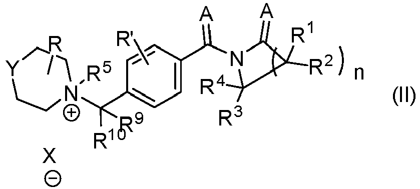 in which R', R⁹ and R¹⁰ independently of one another represent H, a C₁₋₃₀ alkyl, C₃₋₃₀ cycloalkyl, aryl, alkylaryl or arylalkyl radical, in which 1 or more non-adjacent carbon atoms not bonded to a heteroatom may be replaced by a heteroatom, in particular N, O, S and/or Se.

## Revendications

1. Composé activateur de blanchiment de formule générale (l), 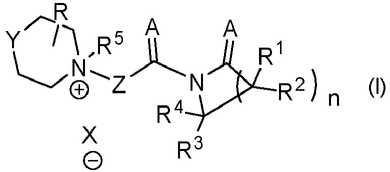 dans la
A représente O, S, Se ;
Z représente un radical alkylène en C₁₋₃₀ , cycloalkylène en C₃₋₃₀ , arylène, alkylène-arylène ou arylène-alkylène, éventuellement substitué ;
X⁻ représente un anion, en particulier un chlorure, un bromure, un iodure, un tosylate, un mésylate, un triflate, un sulfate, un carbo nat et/ou un phosphate ;
Y représente NR⁶, O, S, Se ;
R, R¹, R², R³, R⁴, R⁵ et R⁶ représentent indépendamment les uns des autres H, un radical alkyle en C₁₋₃₀ , cycloalkyle en C₃₋₃₀ , aryle, alkylaryle ou arylalkyle ; et
n représente un nombre compris entre 1 et 12,
où, dans les radicaux hydrocarbonés, 1 ou plusieurs atomes de carbone non voisins, non liés à un hétéroatome ro, peuvent être remplacés par un hétéroatome, en particulier N, O, S et/ou Se.

2. Utilisation de composés de formule générale (l), 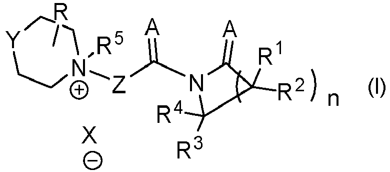 dans la
A représente O, S, Se ;
Z représente un radical alkylène en C₁₋₃₀ , cycloalkylène en C₃₋₃₀ , arylène, alkylène-arylène ou arylène-alkylène, éventuellement substitué ;
X⁻ représente un anion, en particulier un chlorure, un bromure, un iodure, un tosylate, un mésylate, un triflate, un sulfate, un carbo nat et/ou un phosphate ;
Y représente NR⁶, O, S, Se ;
R, R¹, R², R³, R⁴, R⁵ et R⁶ représentent indépendamment les uns des autres H, un radical alkyle en C₁₋₃₀ , cycloalkyle en C₃₋₃₀ , aryle, alkylaryle ou arylalkyle ; et
n représente un nombre compris entre 1 et 12 ;
où, dans les radicaux hydrocarbonés, 1 ou plusieurs atomes de carbone non voisins, non liés à un hétéroatome ro, peuvent être remplacés par un hétéroatome, en particulier N, O, S et/ou Se,
pour renforcer l'efficacité de nettoyage des produits de lavage ou de nettoyage contenant du peroxygène et en particulier des tensioactifs dans un bain aqueux.

3. Utilisation selon la revendication 2, **caractérisée en ce que** l'on introduit le composé de formule générale (l) dans un bain aqueux, en particulier contenant des agents tensioactifs, qui contient également un composé peroxygéné et le textile à nettoyer ou l'objet à nettoyer à surface dure, l'ordre d'introduction du composé selon la formule générale (l) étant le suivant : , Composé peroxygéné et textile ou objet à surface dure est quelconque, ou applique un bain aqueux, en particulier contenant des tensioactifs, qui contient le composé de formule générale (l) et le composé peroxygéné, sur le textile à nettoyer ou sur la surface dure à nettoyer.

4. Procédé de lavage de linge ou procédé de nettoyage de surfaces dures, pour comprenant les étapes de procédé suivantes
(a) préparation d'un bain aqueux contenant HzOz ou un composé peroxygéné, en particulier inorganique, fournissant HzOz dans l'eau, et un composé selon la formule générale (l), 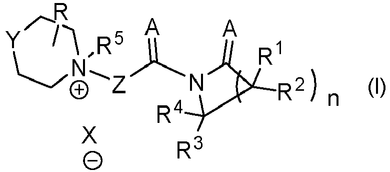 dans la
A représente O, S, Se ;
Z représente un radical alkylène en C₁₋₃₀ , cycloalkylène en C₃₋₃₀ , arylène, alkylène-arylène ou arylène-alkylène, éventuellement substitué ;
X⁻ représente un anion, en particulier un chlorure, un bromure, un iodure, un tosylate, un mésylate, un triflate, un sulfate, un carbo nat et/ou un phosphate ;
Y représente NR⁶, O, S, Se ;
R, R¹, R², R³, R⁴, R⁵ et R⁶ représentent indépendamment les uns des autres H, un radical alkyle en C₁₋₃₀ , cycloalkyle en C₃₋₃₀ , aryle, alkylaryle ou arylalkyle ; et
n représente un nombre compris entre 1 et 12 ;
où, dans les radicaux hydrocarbonés, 1 ou plusieurs atomes de carbone non voisins, non liés à un hétéroatome ro peuvent être remplacés par un hétéroatome, en particulier N, O, S et/ou Se, et
(b) la mise en contact de ce bain avec des textiles à laver ou des surfaces dures à nettoyer.

5. Procédé selon la revendication 4, **caractérisé en ce que** l'on établit le contact entre le bain aqueux et l'objet à laver ou à nettoyer à des températures comprises entre 20°C et 40°C, en particulier entre 20°C et 30°C.

6. Procédé selon la revendication 4 ou 5, **caractérisé en ce que** l'objet à laver ou à nettoyer reste en contact avec le bain aqueux pendant une durée de 20 minutes à 120 minutes, en particulier de 30 minutes à 90 minutes.

7. Utilisation selon la revendication 2 ou 3 ou procédé selon l'une des revendications 4 à 6, **caractérisé en ce que** l'on choisit les quantités de composés peroxygénés à mettre en oeuvre de manière à ce qu'il y ait dans le bain entre 10 ppm et 10 % d'oxygène actif, de préférence entre 50 ppm et 5000 ppm d'oxygène actif.

8. Produit de lavage ou de nettoyage, contenant un composé selon la formule générale (l), 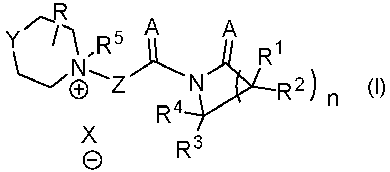 dans la
A représente O, S, Se ;
Z représente un radical alkylène en C₁₋₃₀ , cycloalkylène en C₃₋₃₀ , arylène, alkylène a rylène ou arylène-alkylène, éventuellement substitué ;
X⁻ représente un anion, en particulier un chlorure, un bromure, un iodure, un tosylate, un mésylate, un triflate, un sulfate, un carbo nat et/ou un phosphate ;
Y représente NR⁶, O, S, Se ;
R, R¹, R², R³, R⁴, R⁵ et R⁶ représentent indépendamment les uns des autres H, un radical alkyle en C₁₋₃₀ , cycloalkyle en C₃₋₃₀ , aryle, alkylaryle ou arylalkyle ; et
n représente un nombre compris entre 1 et 12 ;
où, dans les radicaux hydrocarbonés, 1 ou plusieurs atomes de carbone non voisins, non liés à un hétéroatome ro, peuvent être remplacés par un hétéroatome, en particulier N, O, S et/ou Se.

9. Produit selon la revendication 8, **caractérisé en ce qu'**il contient de 0,01 % en poids à 50 % en poids, en particulier de 0,1 % en poids à 25 % en poids de composé selon la formule générale (I).

10. Utilisation, agent ou procédé selon l'une quelconque des revendications précédentes, **caractérisé en ce que** le composé de formule générale (l) correspond à la formule générale (ll), 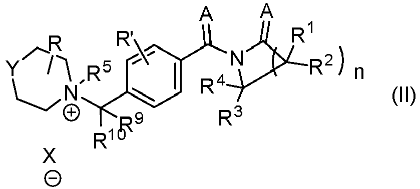 dans laquelle R', R⁹ et R¹⁰ représentent indépendamment les uns des autres H, un radical alkyle en C₁₋₃₀, cycloalkyle en C₃₋₃₀, aryle, alkylaryle ou arylalkyle, dans lesquels 1 ou plusieurs atomes de carbone non voisins, non liés à un hétéroatome, peuvent être remplacés par un hétéroatome, en particulier N, O, S et/ou Se.
